# FASCICULE DE BREVET EUROPEEN

(11) **EP 1 053 033 B1**
(45) Date de publication et mention de la délivrance du brevet: **28.06.2006**
(21) Numéro de dépôt: 99902608.1
(22) Date de dépôt: 05.02.1999
(51) Int. Cl.: A61M 1/02

(54) **PROCEDE DE TRANSFORMATION DE PLASMA ET DISPOSITIF POUR SA MISE EN OEUVRE**
VERFAHREN UND VORRICHTUNG ZUM TRANSFORMIEREN VON PLASMA
METHOD FOR TRANSFORMING PLASMA AND IMPLEMENTING DEVICE

(30) Priorité: 05.02.1998 FR 9801570
(43) Date de publication de la demande: 22.11.2000
(73) Titulaire: MACO PHARMA, F-59200 Tourcoing (Nord) (FR)
(72) Inventeur: DALLE, Valéry, F-59200 Tourcoing (FR); VERPOORT, Thierry, F-59200 Tourcoing (FR); GOUDALIEZ, Francis, F-59200 Tourcoing (FR)
(74) Mandataire: Breese Derambure Majerowicz
(86) Numéro de dépôt international: PCT/FR1999/000260
(87) Numéro de publication internationale: WO 1999/039758

(56) Documents cités:
- EP-A- 0 333 119
- EP-A- 0 933 090
- WO-A-96/32178
- WO-A-97/22245
- DE-U- 29 801 590
- FR-A- 2 677 883
- GB-A- 2 246 713

## Description

La présente invention concerne un procédé de transformation de plasma ainsi qu'un dispositif pour sa mise en oeuvre.

Elle trouvera ses applications dans tous les domaines de l'activité économique dans lesquels on est amené à préparer, traiter et/ou utiliser du plasma issu de prélèvements sanguins, effectués sur l'être humain ou animal.

Actuellement, on emploie le plasma sanguin, notamment, sous forme de dérivés stables et/ou dans la réalisation de produits thérapeutiques.

Pour de telles applications, il est donc nécessaire de disposer de plasma qui permette d'éliminer tous risques de contamination, notamment virale, à partir du ou des donneurs.

Il est ainsi connu, selon une opération appelée déleucocytation, d'éliminer les globules blancs ou leucocytes des prélèvements sanguins réalisés. En effet, il a été établi que, parmi les composants du sang, les leucocytes sont ceux qui présentent une des charges virales les plus importantes. Ils sont également susceptibles de constituer des vecteurs de réplication de certains virus.

Pour déleucocyter, actuellement, on filtre directement le prélèvement sanguin total réalisé. Toutefois, pour éviter un colmatage des éléments cellulaires tels que leucocytes, globules rouges et/ou plaquettes, du prélèvement sanguin sur les parois du filtre, les performances du filtrage sont nécessairement limitées.

Le taux résiduel de leucocytes après filtration peut ne pas être nul et il est encore nécessaire d'opérer différents traitements ce qui complique la préparation du plasma.

De plus, ces traitements postérieurs n'ont été établis que pour des virus connus. Ils ne permettent donc pas de s'assurer de l'élimination de l'ensemble des virus, notamment des virus non identifiés.

En particulier, la plupart de ces traitements ont un effet externe et ne permettent pas d'agir contre les virus contenus dans les leucocytes, ceux-ci pouvant alors être libérés ultérieurement.

Il est connu par ailleurs, notamment du document WO-A-37/22245, un procédé de congélation et de décongélation de plasma provenant d'un prélèvement sanguin. Après décongélation, le plasma est filtré.

Un inconvénient d'un tel procédé est qu'il risque d'entraîner une dispersion des virus dans le plasma ce qui est contraire au but recherché. En effet, lors de la congélation, certains des leucocytes peuvent être endommagés et les virus qu'ils renferment sont susceptibles de se répandre après décongélation.

N'étant plus retenus par l'intermédiaire des leucocytes dans lesquels ils se trouvaient, ils peuvent alors passer la barrière du filtre sans qu'il soit possible de les arrêter.

En outre, la congélation risque d'entraîner la libération d'autres produits indésirables.

Le but de la présente invention est de proposer un procédé et un dispositif de transformation de plasma qui pallient les inconvénients précités et permettent d'améliorer sa déleucocytation.

Un autre but de la présente invention est de proposer un procédé et un dispositif de transformation de plasma dans lesquels les risques de colmatage soient évités.

Un autre but de la présente invention est de proposer un procédé et un dispositif de transformation de plasma qui permettent de respecter les contraintes de stérilisation.

D'autres buts et avantages de l'invention apparaîtront au cours de la description qui va suivre qui n'est donnée qu'à titre indicatif et qui n'a pas pour but de la limiter.

On connaît du document FR-A-2 677 883 un ensemble de poches pour prélèvement de sang comprenant une poche filtrante destinée à permettre la filtration du sang total ou des composants sanguins labiles pour en séparer les globules blancs. Toutefois, ce document ne divulgue pas un ensemble ni un procédé d'utilisation d'un tel ensemble pour la transformation du plasma en circuit clos.

Le document EP-A1-0 933 090 fait partie de l'état de la technique opposable au titre de l'article 54(3) CBE. Ce document décrit un procédé de transformation du plasma dans lequel on extrait le plasma des autres composants avec addition d'une substance d'inactivation pathogène, et un dispositif pour la mise en oeuvre d'un tel procédé qui comprend un moyen d'addition d'une substance d'inactivation pathogène. Les revendications 1 et 5 du brevet contiennent un disclaimer qui est défini par rapport au contenu du document EP-A1-0 933 090.

La présente invention concerne un procédé de de plasma selon la revendication 1.

La présente invention concerne également un dispositif pour la mise en oeuvre du procédé évoqué ci-dessus selon la revendication 5.

L'invention sera mieux comprise à la lecture de la description suivante accompagnée des dessins en annexe qui en font partie intégrante et parmi lesquels :
- la figure 1 illustre un exemple de réalisation du dispositif de transformation de plasma conforme à l'invention,
- la figure 2 détaille un exemple de réalisation d'un des éléments du dispositif de transformation de plasma conforme à l'invention.

La présente invention concerne un procédé de transformation de plasma. Elle trouvera ses applications dans tous les domaines de l'activité économique dans lesquels on est amené à préparer, traiter et/ou à utiliser du plasma issu d'un prélèvement sanguin d'un être humain ou animal.

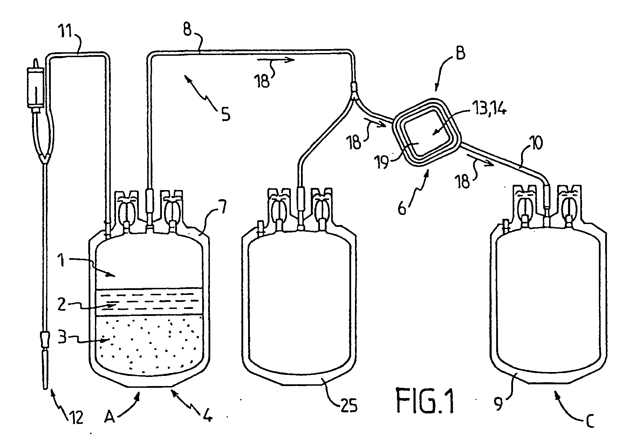

Comme représenté à la figure 1, selon le procédé conforme à l'invention, on sépare tout d'abord les différents composants d'un prélèvement sanguin 1, par exemple au niveau d'une zone repérée A. On effectue une telle opération, notamment, par centrifugation.

De manière connue, ledit prélèvement 1 est alors séparé, notamment en trois couches, une couche supérieure 1 constituée du plasma, une couche médiane 2 constituée des leucocytes et des plaquettes et/ou une couche inférieure 3 constituée des globules rouges.

On extrait ensuite le plasma 1 des autres composants et on le filtre, notamment dans une zone B pour le déleucocyter, le plasma étant toujours sous forme de plasma frais, liquide, natif lors de cette étape.

Le fait, selon l'invention, de filtrer le plasma seul et sous forme native, c'est-à-dire après séparation de celui-ci des autres composants du prélèvement sanguin mais sans l'avoir congelé entretemps permet de diminuer le taux d'éléments cellulaires, tels que leucocytes, à filtrer et d'améliorer ainsi la déleucocytation En effet, seuls ceux éventuellement entraînés lors de l'extraction auront à être retenus lors du filtrage. En outre, on évite la libération d'organismes indésirés.

Après filtration, on recueille le plasma obtenu, notamment dans une zone C. Naturellement, le plasma filtré pourrait encore, éventuellement, être traité, notamment afin d'éliminer les virus étrangers aux leucocytes.

Selon un mode de réalisation avantageux de l'invention, on filtre le plasma 1 extrait à l'aide d'un filtre écran, c'est-à-dire à l'aide d'un filtre ne laissant passer que les particules de taille inférieure à celles des leucocytes.

Selon les procédés antérieurement utilisés, un tel filtre aurait entraîné automatiquement un colmatage compte tenu du fort taux de concentration d'éléments cellulaires de grosse taille dans un prélèvement sanguin total ce qui n'est plus le cas avec l'invention, seul le plasma extrait desdits prélèvements après séparation étant filtré.

Cela étant, il est également possible, de filtrer en outre le plasma 1 extrait à l'aide d'un filtre en profondeur, c'est-à-dire à l'aide d'un filtre dans lequel les éléments cellulaires sont arrêtés au fur et à mesure de la progression du liquide dans le filtre, ceci en plusieurs étapes.

Selon ce mode particulier de réalisation, on réalise ladite filtration à l'aide du filtre écran sensiblement à la fin de ladite filtration à l'aide du filtre en profondeur.

On pourra ainsi filtrer le plasma extrait à l'aide du filtre écran, notamment, lors des dernières étapes du filtrage en profondeur.

Comme développé plus loin, on réalise les différentes étapes de séparation, extraction et filtration, ainsi qu'une éventuelle étape initiale de collecte du prélèvement sanguin dans un circuit clos. En se reportant de nouveau à la figure 1, on constate que le dispositif pour la mise en oeuvre du procédé conforme à l'invention comprend au moins des moyens 4 pour permettre la séparation des différents composants d'un prélèvement sanguin, des moyens 5 pour permettre l'extraction du plasma 1 des autres composants 2, 3 et des moyens 6 de filtration du plasma 1 extrait.

Lesdits moyens 4 pour permettre la séparation sont constitués, par exemple, d'une poche de traitement 7, apte à subir notamment une centrifugation.

Les moyens 5 pour permettre l'extraction comprennent, notamment, un conduit 8 relié à l'une de ses extrémités à ladite poche de traitement 7 et, à l'autre, aux moyens pour filtrer 6.

En aval de ces derniers, une poche de recueil 9 pourra éventuellement être prévue. Elle est reliée auxdits moyens pour filtrer 6 par l'intermédiaire d'un conduit 10.

Le dispositif conforme à l'invention comprend en outre, par exemple, un conduit 11 reliant ladite poche de traitement 7 à des moyens 12 pour collecter le prélèvement sanguin total.

Selon un mode avantageux de l'invention, lesdits moyens 6 pour filtrer comprennent au moins un filtre écran 13. Il est constitué, par exemple, d'une grille poreuse, dont les pores sont inférieurs à 7µm.

Lesdits moyens 6 pour filtrer comprennent en outre, éventuellement, un filtre en profondeur 14.

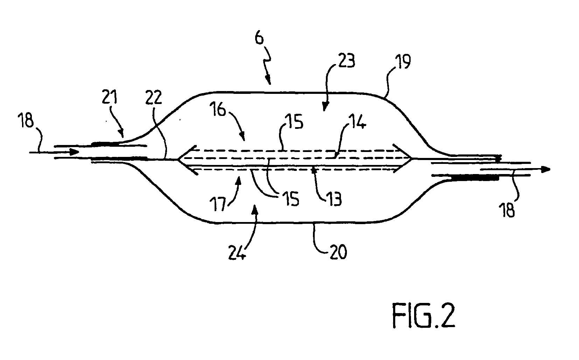

Comme représenté à la figure 2 ledit filtre en profondeur 14 est constitué, par exemple, d'une masse de feuillets filtrants 15, et présente une face d'entrée 16 et une face de sortie 17.

La notion de faces d'entrée 16 et de sortie 17 est définie par le sens de circulation du plasma dans les moyens pour filtrer 6, illustré par les flèches repérées 18.

Lesdits feuillets filtrants 15 sont constitués, par exemple, de non-tissés dont la taille des pores est définie pour chaque feuillet. Ceux-ci présentent ainsi, notamment, une taille moyenne de pores décroissante de la face d'entrée 16 à la face de sortie 17. Quant à la taille des pores du filtre écran 13, elle est inférieure à celle des feuillets filtrants 15. -

Ledit filtre écran 13 est, par exemple, prévu intercalé dans la masse desdits feuillets filtrants 15, notamment au voisinage de la face de sortie 17 du filtre en profondeur 14. Il est ainsi protégé.

Lesdits moyens pour filtrer 6 se présentent, par exemple, sous la forme d'une enveloppe extérieure souple formée de deux feuilles 19, 20 assemblées sur leur périphérie 21 renfermant ledit filtre en profondeur 14 et ledit filtre écran 13.

Ceux-ci sont maintenus, notamment, dans un cadre 22 souple étanche qui délimite deux compartiments 23, 24 respectivement d'entrée et de sortie des moyens pour filtrer 6 qui constituent ainsi une poche filtrante.

Le compartiment de sortie 24 est, par exemple, dégagé du filtre en profondeur 14 et/ou du filtre écran 13 par la présence de joncs d'écartement souples, non illustrés.

Cela étant, en se reportant de nouveau à la figure 1, on constate que le dispositif conforme à l'invention pourra également contenir, par exemple, une poche de recueil des globules rouges 25, notamment préremplie d'une solution de SAGM.

Comme précédemment évoqué, le dispositif conforme à l'invention est utilisé en circuit fermé. Les moyens pour permettre la séparation 4, les moyens pour permettre l'extraction 5 et les moyens pour filtrer 6 sont alors préconnectés ainsi que, éventuellement, les moyens pour collecter 12, le conduit 11 de raccordement de ceux-ci aux moyens pour séparer 4, la poche de recueil 9 et/ou le conduit de raccordement de celle-ci aux moyens pour filtrer 6. La figure 1 illustre un exemple de mise en oeuvre du dispositif conforme à l'invention suivant un tel mode de réalisation.

L'ensemble des éléments du dispositif conforme à l'invention est réalisé, par exemple, en un matériau souple tel que, notamment, du PVC. Ceci facilite ainsi la centrifugation, voire son conditionnement, notamment dans des poches souples.

Il pourrait néanmoins être prévu d'utiliser un filtre écran 13 et/ou un filtre en profondeur 14 montré sur un support rigide.

L'ensemble des constituants du dispositif conforme à l'invention est, par exemple, stérilisé avant utilisation et/ou conditionné dans une pochette stérilisée, notamment à la vapeur.

Naturellement, d'autres modes de mise en oeuvre, à la portée de l'homme de l'art, auraient pu être envisagés sans pour autant sortir du cadre de l'invention.

## Revendications

1. Procédé de transformation de plasma, dans lequel :
- on sépare les différents composants du prélèvement sanguin (1, 2, 3);
- on extrait le plasma (1) des autres composants (2,3) sans addition d'une substance d'inactivation pathogène;
- on filtre le plasma (1) extrait pour le déleucocyter,
ledit procédé étant **caractérisé en ce qu'**il est réalisé en circuit clos.

2. Procédé selon la revendication 1, dans lequel on filtre le plasma (1) extrait à l'aide d'un filtre écran.

3. Procédé sur la revendication 2, dans lequel on filtre en outre le plasma (1) extrait à l'aide d'un filtre en profondeur.

4. Procédé sur la revendication 3, dans lequel on réalise ladite filtration à l'aide d'un filtre écran à la fin de ladite filtration à l'aide du filtre en profondeur.

5. Dispositif pour la mise en oeuvre du procédé selon la revendication 1, comprenant au moins des moyens de prélèvement (12) du sang total, des moyens (4) pour permettre la séparation des différents composants du prélèvement sanguin (1,2,3), des moyens (5) pour permettre l'extraction du plasma (1) des autres composants (2, 3), des moyens (6) de filtration du plasma (1) extrait et des moyens (9) de recueil du plasma filtré, ledit dispositif étant **caractérisé en ce qu'**au moins les moyens (4) pour permettre la séparation des différents composants du prélèvement sanguin (1, 2, 3), les moyens (5) pour permettre l'extraction du plasma (1) des autres composants (2, 3) et les moyens (6) de filtration du plasma (1) extrait sont reliés de sorte à former un circuit clos ledit dispositif étant dépourvu de moyen d'addition d'une substance d'inactivation pathogène.

6. Dispositif selon la revendication 5, dans lequel lesdits moyens (6) pour filtrer comprennent au moins un filtre écran (13).

7. Dispositif selon la revendication 6, dans lequel lesdits moyens (6) pour filtrer comprennent en outre un filtre en profondeur (14).

8. Dispositif selon la revendication 7, dans lequel ledit filtre en profondeur (14) est constitué d'une masse de feuillets filtrants (15) et présente une face d'entrée (16) et une face de sortie (17).

9. Dispositif selon la revendication 8, dans lequel ledit filtre écran (13) est prévu intercalé dans la masse desdits feuillets filtrants (15) du côté de la face de sortie (17) dudit filtre en profondeur (14).

## Claims

1. Method of transforming plasma, in which:
- the various components of the blood sample (1, 2, 3) are separated;
- the plasma (1) is extracted from the other components (2, 3) without adding a pathogenic inactivation substance;
- the extracted plasma (1) is filtered such as to leukodeplete it,
said method being **characterised in that** it is performed in a closed circuit.

2. Method according to claim 1, in which the extracted plasma (1) is filtered with the help of a gauze screen.

3. Method according to claim 2, in which the extracted plasma (1) is also filtered with the help of a thorough filter.

4. Method according to claim 3, in which said filtering is conducted with the help of a gauze screen at the end of said filtering with the help of a thorough filter.

5. Device for implementing the method according to claim 1, comprising at least means for sampling (12) of whole blood, means (4) that enable the separation of the various components of the blood sample (1, 2, 3), means (5) for enabling the extraction of the plasma (1) from the other components (2, 3), means (6) for filtering the extracted plasma (1) and means (9) for collecting the filtered plasma, said device being **characterised in that** at least the means (4) that enable the separation of the various components of the blood sample (1, 2, 3), the means (5) for enabling the extraction of the plasma (1) from the other components (2, 3) and the means (6) for filtering the extracted plasma (1) are hooked up such as to form a closed circuit, said device being unequipped with means for adding a pathogenic inactivation substance.

6. Device according to claim 5, in which said filtering means (6) comprise at least one gauze screen (13).

7. Device according to claim 6, in which said filtering means (6) also comprise a thorough filter (14).

8. Device according to claim 7, in which said thorough filter (14) consists of a mass of filtering layers (15) and has an input side (16) and an output side (17).

9. Device according to claim 8, in which said gauze screen (13) is provided inserted in the mass of said filtering layers (15) on the output side (17) of said thorough filter (14).

## Patentansprüche

1. Verfahren für die Transformation von Plasma, bei dem man:
- die einzelnen Bestandteile der Blutprobe (1, 2, 3) trennt;
- das Plasma (1) ohne Zugabe einer pathogenen Inaktivierungssubstanz aus den anderen Bestandteilen (2, 3) abscheidet;
- das abgeschiedene Plasma (1) filtert, um es zu deleukocytieren,
wobei das besagte Verfahren **dadurch gekennzeichnet ist, dass** es im geschlossenen Kreis durchgeführt wird.

2. Verfahren nach Anspruch 1, bei dem man das abgeschiedene Plasma (1) anhand eines Blendenfilters filtert.

3. Verfahren nach Anspruch 2, bei dem man das abgeschiedene Plasma (1) ferner anhand eines Tieferfilters filtert.

4. Verfahren nach Anspruch 3, bei dem man die besagte Filterung anhand eines Blendenfilters am Ende der besagten Filterung anhand des Tiefenfilters durchführt.

5. Vorrichtung für die Umsetzung des Verfahrens nach Anspruch 1, mit zumindest Probeentnahmemitteln (12) des gesamten Bluts, Mitteln (4) für die Trennung der einzelnen Bestandteile der Blutprobe (1, 2, 3), Mitteln (5) für die Abscheidung des Plasmas (1) aus den anderen Bestandteilen (2, 3), Mitteln (6) für die Filterung des abgeschiedenen Plasmas (1) und Mitteln (9) zum Auffangen des gefilterten Plasmas, wobei die besagte Vorrichtung **dadurch gekennzeichnet ist, dass** zumindest die Mittel (4) für die Trennung der einzelnen Bestandteile der Blutprobe (1, 2, 3), die Mittel (5) für die Abscheidung des Plasmas (1) aus den anderen Bestandteilen (2, 3) und die Mittel (6) für die Filterung des abgeschiedenen Plasmas (1) so miteinander verbunden sind, dass sie einen geschlossenen Kreis bilden, wobei die Vorrichtung kein Zugabemittel einer pathogenen Inaktivierungssubstanz umfassen.

6. Vorrichtung nach Anspruch 5, bei der die besagten Mittel (6) für die Filterung mindestens einen Blendenfilter (13) umfassen.

7. Vorrichtung nach Anspruch 6, bei der die besagten Mittel (6) für die Filterung ferner einen Tiefenfilter (14) umfassen.

8. Vorrichtung nach Anspruch 7, bei der der Tiefenfilter (14) aus einer Masse von Filterblättern (15) besteht und eine Eingangsfläche (16) sowie eine Ausgangsfläche (17) aufweist.

9. Vorrichtung nach Anspruch 8, bei der der besagte Blendenfilter (13) auf der Seite der Ausgangsfläche (17) des besagten Tiefenfilters (14) in die Masse der besagten Filterblätter (15) eingelassen ist.
